# EUROPEAN PATENT SPECIFICATION

(11) **EP 0 460 016 B1**
(45) Date of publication and mention of the grant of the patent: **15.12.1993**
(21) Application number: 90903555.2
(22) Date of filing: 29.01.1990
(51) Int. Cl.: C12N 9/64, C12Q 1/37

(54) **METASTASIS-ASSOCIATED COLLAGENOLYTIC METALLOPROTEINASES**
METASTASENZUGEHÖRIGE KOLLAGENOLYTISCHE METALLOPROTEINASE
METALLOPROTEINASES COLLAGENOLYTIQUE ASSOCIEES A LA METASTASE

(30) Priority: 27.02.1989 US 316260
(43) Date of publication of application: 11.12.1991
(73) Proprietor: BOARD OF REGENTS, THE UNIVERSITY OF TEXAS SYSTEM, Austin, Texas 78701 (US)
(72) Inventor: NAKAJIMA, Motowa, Houston, TX 77035 (US); NICOLSON, Garth, L., Kingwood, TX 77339 (US)
(74) Representative: Dost, Wolfgang, Dr.rer.nat., Dipl.-Chem.
(86) International application number: US9000506
(87) International publication number: WO9010062

(56) References cited:
- BIOCHEMICAL AND BIOPHYSICAL RESEARCH COMMUNICATIONA, vol. 154, no.3, 1988, M. BALLIN et al."Ras Oncogene Mediated Induction of a 92kDa Metalloproteinase, Strong Correlation with the Malignant Phenotype", pp. 832-838
- ThE CANCER BULLETIN, vol. 39, no.3, 1987, M. NAKAJIMA et al."Basement Membrane Degradative Enzymes and Tumor Metastasis", pp. 142-149
- NATURE, vol. 284, 1980, L.A. LIOTTA et al."Metastatic potential correlates with enzymatic degradation of basement membrane collagen", pp. 67-68
- CHEMICAL ABSTRACTS, vol. 109, no.11, 12 September 1988 (Columbus, Ohio, US), TSUDA, M. et al "High molecular mass type IV collagen-specific metalloprotease from human carcinoma tissue", p. 295, abstract 88532c
- CHEMICAL ABSTRACTS, vol. 111, no.23, 4 December 1989 (Columbus, Ohio, US), p. 448, abstract 212922n, L.A. LIOTTA et al.
- CHEMICAL ABSTRACTS, vol.111, no.23, 4 December 1989 (Columbus, Ohio, US) NETHERY, A. et al."Identification, partial purification and characterization of high-molecular-weight gelatin-degrading metalloproteinases produced by a rat mammary carcinoma cell line", p. 419, abstract 212652z
- CHEMICAL ABSTRACTS, vol. 110, no.15, 10 April 1989 (Columbus, Ohio, US) NETHERY, A. et al."Identification of a metalloporteinase copurifying with rat tumor collagenase and the characteristics of fragments of both enzymes", p. 317, abstract 131003r

## Description

The government may own certain rights in the present invention pursuant to NIH grant numbers RO1-CA41524 and R35-CA44352.

The present invention relates to metastasis-associated collagenolytic metalloproteinases, and their detection as a means for testing for metastatic or recurrent forms of cancers. In particular, the invention relates to a gelatinolytic and type IV collagenolytic metalloproteinase enzyme having a molecular weight on the order of about 88 to 92 kilodaltons, found to be associated with highly metastatic and other forms of cancer cells.

Successful malignant cell penetration of basement membranes is an important step in the formation of tumor metastases. Basement membranes are barriers between different tissues that are rigid structures formed from unique macromolecules, such as type IV collagen, laminin, heparan sulfate proteoglycan, and fibronectin. Several proteinases have been implicated in the process of tumor invasion and metastasis, and some are known to be involved in the degradation of basement membrane components (1,2).

Liotta and coworkers (3) described a type IV collagenolytic proteinase in metastatic tumor cells, and they found a correlation between type IV collagenase activity and metastatic potential (4). Moreover, previous studies authored by the present inventors have indicated a correlation between the type IV collagenolytic activities of rat 13762NF mammary adenocarcinoma cells and their spontaneous lung metastatic potentials (5). The metalloproteinase secreted from rat mammary adenocarcinoma cells was found to degrade both alpha-subunits of type IV procollagen and produce characteristic large molecular weight (Mᵣ) fragments (5).

Type IV collagenases have been purified from a variety of mammalian cells, such as metastatic mouse sarcoma cells (6), human monocytes/macrophages (7), and human H-ras oncogene-transformed bronchial epithelial cells (8). These enzymes are metalloproteinases of Mᵣ 62,000-66,000 in active forms and 66,000-72,000 in latent forms, and some have been found to have gelatinase activity (8). Latent enzymes have been found to be activated by either trypsin digestion or 4-aminophenylmercuric acetate treatment in vitro.

The elevation in expression of certain serum proteins, such as carcinoembryonic antigen, alpha-feto-protein, and placenta-like alkaline phosphatase, is associated with some human cancers and has been used diagnostically for neoplastic disease (9). In certain spontaneously metastasizing tumors, an association between increased tissue glycosyltransferase levels and metastasis formation was found, and high levels of glycosyl transferases have been detected in the sera of animals bearing spontaneously metastasizing tumors (9).

Prior to now, though, there has been no clear functional relationship, between elevated serum levels of such components and metastatic disease. Since tissue-degrading enzymes are secreted in high amounts from invasive tumor cells, the levels of degradative enzymes in body fluids should be useful as diagnostic markers of tumor invasion and metastasis. For example, high levels of beta-N-acetylglucosaminidase and beta-glucuronidase were found in the sera of animals and patients with various types of tumors (9). The association of high levels of serum cathepsin B1-like activity with invasive cancer, such as carcinomas of the ovary, vagina, cervix, and breast, has also been reported (10).

Ballin et al. (Biochemical and Biophysical Research Communications, Vol. 154, No. 3, pages 832-838 (1988)) describe ras-mediated induction of a 92 kDa metalloproteinase, which degrades gelatin and type IV collagen. Association of the 92 kDa proteinase expression with the malignant phenotype has been observed in human tumor cell lines. The data indicate that the 92 kDa gelatin-collagen IV degrading metalloproteinase is an important participant in the protolytic process involving tumor cell invasion and metastasis in humans.

The present invention relates to a novel rat metalloproteinase, having a Mᵣ of about 88-92 kilodaltons, that is capable of specifically degrading type IV collagen and gelatin. An important aspect of this metalloproteinase centers on the inventors' finding that its presence is correlated with certain forms of metastatic cancer. The inventors have thus found that the enzyme can form the basis of diagnostic and monitoring assays. The 88/92 kd metalloproteinase has been found to be associated predominantly with highly malignant mammary tumors, breast cancers, colon cancers and malignant melanomas.

The enzyme itself is characterized as a gelatinolytic and type IV collagenolytic metalloproteinase based on its ability to specifically degrade protein substrates including type IV collagen, type I collagen and gelatin. However, this enzyme has not been found to be active in digesting substrates such as fibronectin, albumin, casein, immunoglobulin, or hemoglobin, suggesting that the range of susceptible molecules might be limited. This proteinase is termed a metalloproteinase due to its requirement for a metal ion for activity. For this reason, the enzyme is found to be inactive in the presence of inhibiting concentrations of chelating agents, such as 10 mM ethylenediaminetetraacetic acid (EDTA) or 1, 10-phenanthroline. However, the enzyme is not inhibited by common proteinase inhibitors such as phenylmethylsulfonyl fluoride (PMSF) at 2 mM or N-ethyl-maleimide (NEM) at 5 mM.

The molecular weight range arrived at for this enzyme, about 88 to 92 kilodaltons, has been determined using sodium dodecyl sulfate-polyacrylamide gel electrophoresis (SDS-PAGE) in conjunction with a technique known as zymography. Zymography involves identifying the location of an enzymatic activity in a gel or similar matrix by providing a substrate for the enzyme, -- a substrate whose interaction with the active enzyme can be detected. The enzyme is in this manner directly "visualized" in the gel or matrix. Zymography is a technique which can be used in the practice of the diagnostic method of the invention, in identifying the enzyme.

The diagnostic method of the invention involves a test for human neoplastic disease, particularly metastatic disease and tumor recurrence, in a patient suspected of having such a disease. The method includes generally testing for the presence of the metalloproteinase in a clinical samples obtained from the patient, such as serum, tissue samples, aspirates, and other body fluids, but preferably serum. The particular technique employed to detect the metalloproteinase will generally be based on characteristics of the enzyme, ranging, for example, from enzymatic characteristics and molecular weight, probed by zymography, to its primary structure that can be probed immunologically.

In diagnostic methods based on zymography, a clinical sample suspected of containing the enzyme is subjected to electrophoresis in a substrate-embedded gel to separate proteins within the sample. The substrate employed for identification of the metalloproteinase will typically be type I or IV collagen, gelatin, or some other suitable substrate the enzyme may be found active upon. In the practice of a zymography-related embodiment, one will generally desire to separate the proteins according to their molecular weights. This allows one to identify the metalloproteinase of the invention according to its molecular weight, at the same time as determining its relative collagenolytic and/or gelatinolytic enzyme activity.

After electrophoresis, the gel is incubated under conditions appropriate to allow any metalloproteinase which may be present in the gel to lyse the substrate. Where one has fractionated the proteins according to molecular weight, it will generally be the case that an SDS-PAGE system of fractionation will have been employed. Under these circumstances, where enzymatic activity is to be detected, one will desire to renature the enzyme, in situ because sodium dodecryl sulfate (SDS) tends to denature proteins and reduce or destroy enzymatic activity. Suitable renaturation can be achieved by rinsing the gel in a dilute nonanionic detergent such as 2.5% Triton X-100, followed by incubation for several hours in an isotonic buffer, such as one containing Tris-HCl and saline in the presence of calcium chloride. Staining of the substrate remaining in the renatured incubated gel, such as through the use of a dye like Coomassie blue, allows one to visualize the enzyme indirectly by means of the appearance of a transparent band against a stained background.

Although one can generally identify the presence of the metalloproteinase of the invention by reference to the relative migration of molecular weight markers, it will often be desirable to employ a metalloproteinase standard. This allows a direct comparison of a suspected activity with that of a known control, thus improving the overall reliability and sensitivity of the assay. In these situations, one may desire to employ an at least partially purified preparation of the metalloproteinase, for example, isolated by techniques developed by the inventors and disclosed herein.

Alternative to the use of a zymographic approach to diagnoses based on identification of the metalloproteinase enzyme activity/size, the present invention contemplates the use of immunologic approaches. Since immunologic approaches do not require the detection of an enzyme activity, they may ultimately prove more advantageous method than zymography. The immunologic methods contemplated include the use of an antibody, such as a monoclonal antibody, having immunospecificity for the metalloproteinase. Such an antibody can readily be prepared, through the use of a purified preparation of the enzyme, by techniques known in the art, or preferably, techniques found to work well in connection with the development of antibodies against other metastasis-related proteins, such as described in USSN 846,938, incorporated herein by reference.

Suitable immunological techniques would include those which employ a known antibody to detect the presence of an unknown antigen in a clinical sample, whether it be a tissue, cell, aqueous sample or the like, thought to contain the identifying enzyme. It is therefore contemplated that techniques such as ELISA, RIA, Western blot, dot blot, dip stick, ELISA inhibition test, and the like, will prove to be useful techniques for identifying the presence of the enzyme in suspected samples.

The metalloproteinase of the invention has been found to be present in rat. While it is not clear at present the degree of overall similarity of the enzyme from the rat and from human, for example, at the amino acid sequence level, there appears to be sufficient similarity for them to be considered functionally equivalent within their respective cells. This enzyme likely constitutes a feature of the requirement of certain tumor cells, such as metastatic cells, to penetrate basement membranes. Its presence in such cells, thus, apparently crosses species. It is proposed that for the purposes of the invention, e.g., use as a control marker for metastasis, enzymes of the 88/92 kd metalloproteinases class from differing species can be used. Of course, for most purposes for use in connection with human use or diagnosis, and certainly where one desires to prepare a preferred antibody, one will desire to obtain and employ the human-derived material,

A metalloproteinase in accordance with an aspect of the invention may be obtained from various metastatic rat cells and lines, particularly metastatic mammary carcinoma cells. The inventors have discovered, for example, that highly metastatic clones developed from experimental rat mammary adenocarcinoma lines like the 13762NF system (11), provide very good tumors for eliciting the serum-borne production of the enzyme in a rat host, or even direct production by means of a conditioned media approach. Exemplary metastatic rat mammary adenocarcinoma lines include the MTLn3 and MTF7.T35.3 lines, which were derived by clonal selection from the 13762NF system (12)

In other embodiments, the 88/92 kd metalloproteinase can be isolated from human sources. The enzyme has been found to be present in a variety of human tumor lines, including human breast adenocarcinoma cells (MCF7; e.g. ATCC HTB22), human colon cancer cell lines (e.g., the KM series, references 13, 14), human malignant melanoma cell lines (e.g., Hs294T, ATCC HTB140, and A375, ATCC CRL1619) human renal cell carcinoma lines (SN series, reference 15) various cell lines derived from human teratocarcinoma PA-1 (16), and even human astrocytoma cell lines (17). Techniques are disclosed herein for obtaining preparations of the enzyme from human sources, generally through the generation of tumor cell-conditioned media, in which cells are grown in a media that is essentially free of extrinsically added serum proteins. The enzyme is provided from the cell-conditioned media in a more purified state by techniques disclosed herein.

Diagnostic kits for testing for the 88/92 kd metalloproteinase of the invention are contemplated generally as taking two forms depending on whether they are based on enzymatic properties or not, e.g., using zymography or immunology. Zymography-based kits will include one or more reagents employed in carrying out the underlying zymography, e.g., pre-prepared PAGE gels incorporating enzyme substrates, appropriate molecular weight markers, etc., as well as samples of the 88/92 kd control enzyme.

Immunological-based kits, on the other hand, will typically include an antibody having immunoselective binding capability for the 88/92 kd metastasis-associated enzyme, along with means for detecting antibody/antigen reactions. Such immunoreaction detection means are known in the art, and include, e.g., labels or tags such as enzymes, radioactive ligands, molecular tags, and the like. Often, the detection of a first antibody-antigen reaction may be accompanied by a second antibody, having immunospecificity either for the first antibody, or for second or cumulative epitopic site on the targeted antigen (18). Techniques such as the ABC technique can also be employed. This technique uses unlabeled primary antibody, followed by biotinylated secondary antibody and then a preformed avidin and biotinylated horse radish peroxidase or alkaline phosphatase complex (19).

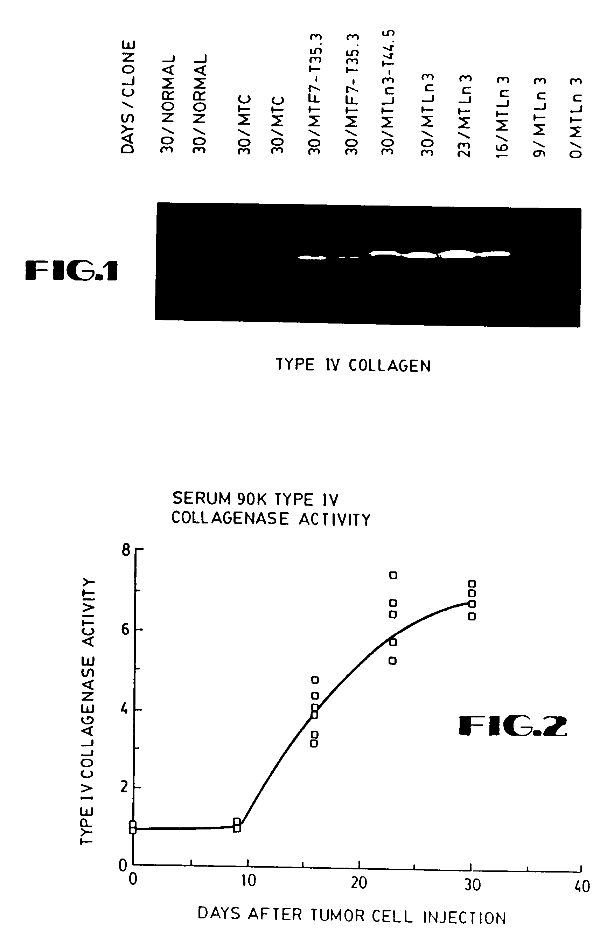

Figure 1. Zymogram of rat serum type IV collagenolytic metalloproteinases. Blood was withdrawn at various periods of time after injection of 13762NF mammary adenocarcinoma clone cells into the mammary fat pad. Clone MTLn3, MTLn3-T44.5, and MTF7-T35.3 cells were highly metastatic to lung and lymph nodes, whereas MTC cells did not spontaneously metastasize, even after 30 days. Serum (2.5 ul) was dissolved in sample buffer containing sodium dodecyl sulfate and subjected to electrophoresis in a 7.5% polyacrylamide gel embedded with 0.5 mg/ml type IV collagen. After electrophoresis the enzymes were renatured by incubation with 2.5% Triton X-100 in 50 mM Tris-HCl, pH 7.5, and the enzyme reaction was performed at 37`C for 16 h in 0.15M NaCl, 10 mM CaCl₂ and 50 mM Tris-HCl, pH 7.5. Proteinases were detected as transparent bands on the blue background of Coomassie blue-stained slab gels. The strained gels were photographed using Kodak electrophoresis duplicating papers.

Figure 2. Type IV collagenase activity in sera of rats bearing MTLn3 mammary adenocarcinoma. Quantitation of the proteinase activity was estimated by photographing the zymograms using Kodak XAR-5 X-ray films. The active enzyme bands that appeared as positively stained bands on the X-ray films were scanned for absorbance at 560 nm. The area under each peak that represents proteolytic activity was measured and shown as a relative enzyme activity.

Figure 3. Relationship between serum Mᵣ 90,000 type IV collagenase activity (in Fisher 344 rats using MTLn3 cells) and number of spontaneous lung metastases. (n=30)

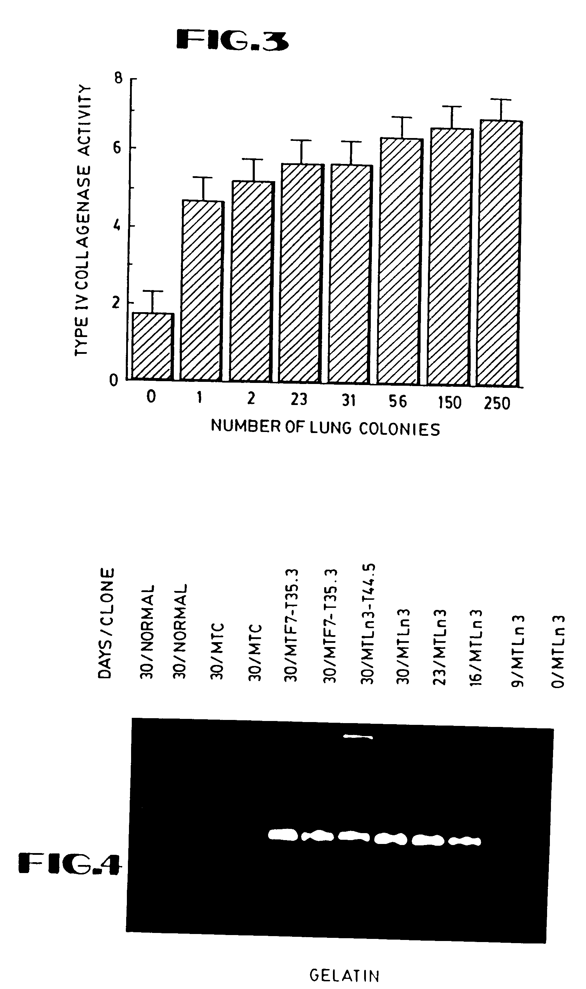

Figure 4. Zymogram of rat serum gelatinolytic metalloproteinases (see Figure 1 legend).

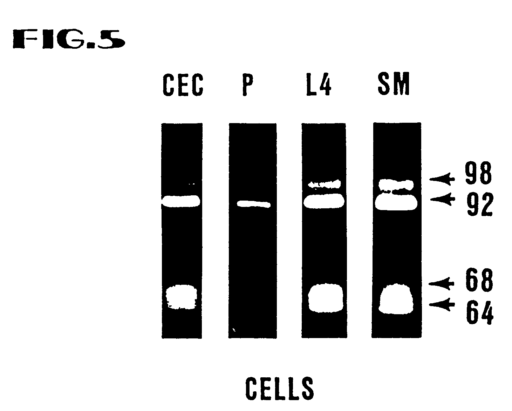

Figure 5. Identification of type IV collagenolytic metalloproteinases secreted by cultured human colon carcinoma cells. Colon carcinoma cells, such as KM12C (C), KM12L1 (L1), KM12L4 (L4), and KM12SM (SM), were cultured for 48 h in a serum-free complete medium, and 2-ml aliquots of conditioned media were concentrated and immediately analyzed by electrophoresis in type IV collagen-embedded polyacrylamide gels followed by 24 h incubation and Coomassie blue staining for detection of in situ type IV collagenolytic activity. Enzymes were detected as transparent bands. Ordinate, molecular weight in thousands.

Metastasis, the process by which cancer cells spread from the primary tumor via the circulation to distant organs, is a complex phenomenon requiring many sequential steps. One of the initial steps is the invasion of primary tumor cells into the surrounding host tissues and blood vessels or lymphatic channels (intravasation). Via the circulation, the tumor cells are disseminated to various organs, where they adhere specifically, or are arrested nonspecifically in capillary beds. Subsequent steps include extravasation by invasion through capillary endothelial cells and the subendothelial ECM³ into the tissues and proliferation of the cells to form secondary tumors. The successful completion of the cascade of events culminating in the formation of metastases depends on the expression of unique properties by progenitor tumor cells, as well as on tumor cell-host interactions (for reviews, see refs. 20-22).

One tumor cell property that is a prerequisite for metastasis is the ability to degrade connective-tissue ECM and basement membrane components, which constitute barriers against invading tumor cells. Indeed, metastatic tumor cells have been shown to produce enzymes such as proteases and glycosidases that are capable of degrading the various components of the ECM (23). One of the proteolytic enzymes, type IV collagenase, which exhibits the ability to degrade type IV collagen, the major structural protein constituent of subendothelial basement membranes, is believed to play an important role in invasion because its levels correlate with the metastatic potential of various transformed and malignant cells (22-23), including rat mammary adenocarcinoma clones derived from the 13762NF cell line (5).

The present invention is directed to a novel, specific rat metalloproteinase found to be associated with metastatic and recurrent neoplastic disease. The metalloproteinase of the invention is characterized as having a molecular weight of on the order of 88 to 92 kilodaltons as determined by SDS-PAGE/zymography. This particular metalloproteinase exhibits both type I and IV collagenolytic activity, as well as gelatinolytic (denatured type I collagen) activity. The enzyme has not been found to exhibit lytic activity with fibronectin, albumin, casein, immunoglobulin, or hemoglobin substrates. These properties suggested that the enzyme is essentially collagen-specific, at least with regard to those substrates which have been tested.

The 88/92 kd metalloproteinase may be readily isolated from either rat or human tumor sources which secrete the enzyme, preferably through the preparation of a conditioned media. As those of skill in the art are aware, conditioned medium is medium which has been employed to grow cells which will contain within it biological materials secreted by the cells. Thus, in the case of the metalloproteinase, growth of cells which produce this enzyme in a selected growth medium result in the secretion of the enzyme into the medium, thus providing a ready source for its isolation. Typically, the media employed for the preparation of a conditioned medium will be free of added serum or serum products. This avoids the introduction of an external source of contamination and impurities presented by growth of cells in serum-containing medium.

For isolation of the metalloproteinase from conditioned media, whether it be from rat or human sources, one will generally desire to first culture the starting cells in serum-containing media for a period of time to allow the cells to begin producing useful amounts of the enzyme. Then, the serum-containing medium is removed and replaced with a serum-free medium. The cells are then allowed to grow on the serum-free medium for a period of time which is selected based on the ability of the cell line or cell source being employed to grow on serum-free medium. This is due to the fact that certain cell lines, and even sublines, tend to grow less well than others under serum-free conditions. This is presumably due to the absence of factors needed for proper growth of cells. It will be understood by those of skill in the art that serum-free growth conditions will likely have to be adjusted depending on the cell line or type used for enzyme production.

After growth of the cells under serum-free conditions for a period of time to allow for secretion of the enzyme into the medium, the medium is harvested and the enzyme present in the medium adsorbed onto a heparin-ligand column, such as a heparin-Sepharose column. It has been found that the 88/92 kd metalloproteinase binds to such a column when adsorbed at under isotonic conditions, and will elute at a salt concentration range of on the order of about 0.3 to 0.5 M salt. Further purification of the enzyme may then be achieved by subsequent chromatography of the heparin-eluted fraction on gel exclusion matrices such as Sephacry S-200 and/or Sepharose CL-6B.

In addition to the obtaining of relatively purified preparations of the 88/92 kd metalloproteinase, the invention further concerns a diagnostic assay for cancer based on the presence of this enzyme in clinical samples. Since the enzyme is found to be present in a variety of human tumors, but not in most non-tumor tissues which have been tested, it is proposed that its presence will be diagnostic of a wide variety of cancers. However, in that the enzyme is apparently associated with particular types of cancer, including in particular metastatic and recurrent forms of cancer, the diagnostic identification of the presence of the enzyme will be particularly useful, for example, in the sera of patients suspected of having such forms of cancer.

One method of identifying the enzyme in samples is through the use of a technique known as zymography, wherein samples suspected of containing the enzyme are allowed to act upon an enzyme substrate in situ, such as in an electrophoretic gel matrix, which has been impregnated with a substrate of the enzyme. In the case of the present invention, the substrate will typically be gelatin or type IV collagen. The enzyme activity can then be identified by the appearance of bands in the matrix where the enzyme has digested or otherwise acted upon the substrate. A number of general methods are known in the art for conducting zymographic analysis of samples, and such methods can be readily adapted for use in connection with the present invention without undue experimentation in light of the specific disclosure herein.

Another method for diagnostic identification of the enzyme in clinical samples is through the use of immunologic techniques, e.g., through the use of an antibody capable of specifically interacting with and thereby identifying the enzyme. Typically this will be achieved through the preparation of a monoclonal antibody having the desired immunospecificity. It is believed that suitable monoclonal antibodies for use in connection with the invention can be prepared using the techniques herein disclosed.

The examples which follow illustrate various preferred embodiments of carrying out the practice of the invention. It will be appreciated by those of skill in the art that various aspects of these examples are conducted through the use of standard laboratory practices of the inventors in conjunction with techniques developed by the inventors for carrying out the invention. For this reason, it will be apparent that modifications and changes can be made in various of the procedures without departing from the intended spirit and scope of the invention.

### EXAMPLE I

### Identification of the 88/92 Metalloproteinase in a Rat Host, through the use of Rat Metastatic Mammary Adenocarcinoma Cells

Highly metastatic clonal lines developed from the rat 13762NF mammary adenocarcinoma system were tested for their ability to elicit the production of the 88/92 metalloproteinase. The appearance of the 88/92 kd enzyme in sera of tumor-burdened animals, or in tumor cell conditioned media, was detected through the use of zymographic techniques, using either type IV collagen or gelatin.

The highly metastatic clones which were to be tested were first injected subcutaneously into the rat mammary fat pad, and allowed to grow. At various time intervals following fat pad injection, serum was obtained from the rat and tested for presence of the 88/92 kd protein by zymography. It was generally found to be the case that the 88/92 metalloproteinase appeared in rat serum approximately 16 or so days following subcutaneous injection. Not only was the activity of this high molecular type IV collagenase in sera found to correlate with the extent of metastasis in lung, but the activity was not detected in the sera of control rats or rats bearing non-metastatic mammary adenocarcinoma cell lines.

In addition to being tested for their ability to elicit the appearance of the 88/92 kd enzyme in host sera, various cells were also tested for their ability to produce the enzyme in conditioned media. Thus, it is clear that the enzyme is a product of the tumor, and not the result of some host/tumor interaction.

The particular metastatic cloned lines employed to illustrate this aspect of the invention are designated MTLn3, MTF7-T35.3 and MTLn3-T44.5. The T35.3 and T44.5 lines are highly metastatic subclones of MTF7 and MTLn3 system derivation, described in reference 12. Also tested were relatively non-metastatic lines such as MTLn2 and MTC.

### CELLS

Rat mammary adenocarcinoma cell clone MTF7-T35.3 was selected by in vitro cloning from MTF7 cells derived from a 13762NF mammary adenocarcinoma tumor growing at a local implant site in the mammary fat pad (12). Clone MTC cells were similarly obtained. The rat mammary adenocarcinoma cell clone MTLn3-T44.5 was cloned in like fashion from MTLn3 cells derived from a 13762NF tumor that spontaneously metastasized from the mammary fat pad to the lung (12).

Cells were grown on 100-mm tissue culture plates (Corning Glass Works, Corning, NY) containing alpha-MEM (GIBCO Laboratories, Grand Island, NY) supplemented with 10% FBS (Biocell, Carson, CA) and no antibiotics at 37`C in a humidified incubator (5% CO₂ and 95% air). In some experiments, MTF7.T35.3 cells were grown in the absence of serum in D-MEM containing 5 ug/ml of insulin, 5 ug/ml of transferrin, 20 uM ethanolamine, 2 mM glutamine, 25 mM selenious acid, 0.2 mM each of 10 nonessential amino acids, and 10 mM N-2-hydroxyethylpiperazine-N'-propanesulfonic acid buffer, pH 7.5 (serum-free complete medium).

### ZYMOGRAMS

Identification of type IV collagenolytic enzymes secreted by mammary adenocarcinoma cells was performed by electrophoresis of serum-free conditioned media in a type IV collagen-embedded polyacrylamide gel followed by incubation and Coomassie blue staining, based on the methods described by Heussen and Dowdle (24). Type IV procollagen was purified from Engelbreth-Holm-Sworn tumors, dissolved in 0.5 M acetic acid or bovine lens capsule type IV collagen pepsin fragments (Seikagaku America, St. Petersburg, FL) in HCl at pH 3.0, neutralized with Tris and immediately dissolved in 2% SDS. After removing undissolved materials by a brief centrifugation at 18,000 x g, SDS-solubilized type IV collagen was copolymerized with 7.5% acrylamide. In some experiments, each of the following proteins was also embedded in SDS-polyacrylamide gels: rat hemoglobin; rat immunoglobulin G; bovine serum ablumin; bovine plasma fibronectin; bovine alpha-casein; and gelatin from swine skin (Sigma Chemical, St. Louis, MO). Electrophoresis of serum, or other samples such as one obtained from conditioned media, was carried out by the method of Laemmli (25) in polyacrylamide gels containing 0.5 to 1.0 mg/ml of type IV collagen.

In the case of serum samples, serum (2.5 ul) was mixed with the same volume of sodium dodecyl sulfate sample buffer without reducing agents, and immediately subjected to electrophoresis in a 7.5% polyacrylamide gel containing the protein substrates. After electrophoresis, gels were rinsed twice with 2.5% Triton X-100 in 50 mM Tris-HCl buffer, pH 7.5, and incubated at 37`C for 16 h in 0.15 M NaCl, 10 mM CaCl₂ and 50 mM Tris-HCl buffer, pH 7.5, containing 0.05% NaN₃. Gels were stained with 0.05% Coomassie blue, 10% isopropanol, and 10% acetic acid in H₂0 and these destained with 10% isopropanol and 10% acetic acid in H₂0. Type IV collagenolytic enzymes were detected as transparent bands on the blue background of Coomassie blue-stained slab gels.

The stained gels were photographed using Kodak electrophoresis duplicating papers (Kodak, Rochester, NY). Quantitative analysis of the collagenolytic enzyme activity was achieved by photographing the zymograms using Kodak X-AR-5 X-ray films. The active enzyme bands that appeared as positively stained bands on the X-ray films were scanned for absorbance at 450 nm using a Beckman DU-8 spectrophotometer. The area under each peak that represents collagenolytic activity was measured and shown as a relative enzyme activity.

In order to conduct zymography using samples obtained by the preparation of conditioned media, the following steps were performed. Mammary adenocarcinoma cells (1 x 10⁶ cells) were seeded in a 10-cm tissue culture plate and cultured for 24 h in serum-free complete medium containing 10% heat-inactivated FBS. The cells were extensively washed with DPBS and then further cultured in 10 ml of serum-free complete medium. The serum-free culture supernatants were withdrawn and sequentially centrifuged at 800 x g and 18,000 x g, and then aliquots of the supernatants were concentrated with Centricon 30 concentrators (Amicon, Danvers, MA) and mixed with SDS sample buffer.

Cell extracts in Triton X-100 were also prepared to examine the total cellular enzyme activities. Cells (1 x 10⁶) were grown as described above, extensively washed with DPBS, and then solubilized in 4`C in 0.2% Triton X-100 in 50 mM Tris-HCl buffer, pH 7.5. Cell extracts were centrifuged for 5 min at 18,000 x g, and the supernatants were immediately mixed with SDS sample buffer without beta-mercaptoethanol.

### Identification of Type IV Collagenolytic Metalloproteinases by Zymograms

When the conditioned media from 48 h cultures were analyzed on zymograms, two major proteinases of apparent molecular weights of 88,000 and 64,000 were detected as clear bands. These active enzyme bands did not appear when a chelating agent, such as EDTA or 1,10-phenanthroline, was added to the incubation buffer at a concentration of 10 mM, whereas phenylmethylsulfonyl fluoride (2 mM) and N-ethylmaleimide (5 mM) did not inhibit the enzyme activity. These results indicate that the Mᵣ 88,000 and 64,000 proteinases are both metalloproteinases. These two proteinase bands were also observed in gels embedded with gelatin or with pepsin fragments of type IV collagen instead of intact type IV collagen. However, when polyacrylamide gels were embedded with serum albumin, hemoglobin, immunoglobulin G, alpha-casein, or fibronectin instead of type IV collagen, the Mᵣ 88,000 and the Mᵣ 64,000 bands did not appear. The results indicated that these enzymes are specific for gelatin and might be responsible for the collagenolytic activity.

The 88/92 kilodalton type IV collagenolytic metalloproteinase was clearly detectable in the sera of rats bearing metastatic mammary adenocarcinoma cell lines, such as MTLn3 and MTF7.T35.3 (Figures 1 and 4). As can be seen from Figure 1, the presence of the 88/92 kd enzyme, using a type IV collagen substrate, was readily apparent in the sera of rats by day 16 following fat pad injection of MTLn3 cells. Similar results were obtained with the other metastatic lines, including MTF7-T35.3 and MTLn3-T44.5. However, no such bands were detectable in the case of the MTC-bearing rats or control rats.

A virtually identical result was observed using a gelatin substrate for the zymographic gels (Figure 4). As can be seen in Figure 4, by 16 days post-injection, sera from the MTLn3 bearing rats exhibited a pronounced band corresponding to the 88/92 kd enzyme, using a gelatin substrate. Moreover, similar to type IV collagen-based experiments no corresponding bands of enzyme activity were seen in sera from normal or MTC-bearing rats.

Type IV collagenase activity levels in sera of rats bearing MTLn3 mammary adenocarcinoma were also measured quantitatively as a function of time (Figure 2). Quantitation of the proteinase activity was estimated by photographing the zymograms using Kodak XAR-5 X-ray films. The active enzyme bands that appeared as positively stained bands on the X-ray films were scanned for absorbance at 560 nm. The area under each peak that represents proteolytic activity was measured and shown as a relative enzyme activity. As can be seen from Figure 2, the enzyme levels began rising after day 10 following fat pad injection, and appeared to peak on about day 30.

Studies were also conducted to identify the correlation, if any, between increased levels of serum high molecular weight (88/92 kd) type IV collagenase activity with the extent of lung metastases (Figure 3). These experiments were performed as follows: MTLn3 cells were harvested from subconfluent cultures by 0.25% trypsin treatment. Rats received 1 X 10⁶ MTLn3 cells/0.5 ml phosphate buffered saline in the left posterior inguinal mammary fat pad. Rats were sacrificed 30 days post-injection of tumor cells using Metofane anestheria and the blood was withdrawn to prepare serum for the enzyme assay. Rats' bodies were subjected to complete gross necropsies. After fixing lung tissues in neutral buffered formalin, the number of lung surface metastases were measured. The relative activity of 88/92 kd type IV collagenase in serum was determined by zymography as described above. As shown in Figure 3, the results demonstrated a direct correlation between the level of type IV collagenase activity that was measured, and the number of spontaneous tumor lung colonies observed by a disecting microscope.

### EXAMPLE II

### Demonstration of the 88/92 kd Type IV Metalloproteinase in Human Tumors

A number of human tumors, including colon carcinoma cell lines, malignant melanoma cell lines, renal cell carcinoma lines, human breast cancer lines, terato-carcinoma cell lines and the sera of malignant breast cancer patients, were tested for the presence of the 88/92 kd metalloproteinase. The cell lines were tested zymographically for the 88/92 kd enzyme through the use of conditioned media, whereas the cancer patients were tested through the use of their sera.

The colon carcinoma lines tested were derived from both primary human colon carcinomas (HCC) (KM12) and KM20 series), as well as from a liver metastasis (KM23 series). The development of these lines is described by Morikawa et al. Highly metastatic subclones were obtained by passage through nude mice and selection. The techniques employed for HCC testing were as follows:

### Mice

Male athymic BALB/c nude mice were obtained from the Animal Production Area, NCI-Frederick Cancer Research Facility (Frederick, MD). Mice were used when 8 weeks old and were maintained in a laminar flow cabinet under specific pathogen-free conditions.

### Tumor Lines

Fresh tumor specimens from primary HCC (KM12:Dukes' B₂ and KM20: Dukes 'D) and from a liver metastases (KM23: Dukes'D) were obtained at surgery from 3 different patients. Tumor tissue was enzymatically dissociated with collagenase (type I, 200 units/ml) and DNase (270 units/ml) (Sigma Chemical Co., St. Louis, MO). The cell suspension was filtered through a 4-layer sterile gauze and washed 3 times in serum-free medium. The procedure yielded a suspension of mainly single tumor cells or small clumps of cells (<5) with a viability of <80% (trypan blue exclusion). Suspensions of 2 x 10⁶ viable cells were established in culture and injected into the subcutis or spleen of different nude mice. Eight to twelve weeks later, the mice were killed. Local growth (tumorigenicity) and distant growth (liver nodules) were determined.

### Selection of Highly Metastatic Cells from the KM12C Line

Subsequent to establishment in culture, cells of the KM12C (Dukes' B) were implanted into the spleen of nude mice. Solidary lesions from the liver (experimental metastases) were isolated and established in vitro (KM12L1 line). This cycle was repeated four times to yield line KM12L4 as described (14). Cells of parental KM12C were also injected into the cecum of nude mice. When the mice became moribund, they were killed, a spontaneous liver metastasis was harvested, established in culture, and designated as KM12SM line. All the lines were shown to be of human origin by karyotype analysis and isoenzyme determinations (Authentikit, Corning Medical, Corning, NY).

### Enzymography

Identification of type IV collagenolytic enzymes secreted by colon carcinoma cells was performed by electrophoresis of serum-free conditioned medium in a type IV collagen-embedded polyacrylamide gel followed by incubation and Coomassie blue staining, according to methods described by Heussen and Dowdle. Bovine corneal type IV collagen (Seikagaku America, St. Petersburg, FL) in HCl was neutralized with Tris, immediately dissolved in 2% sodium dodecyl sulfate, and then copolymerized with 7.5% acrylamide. Colon carcinoma cells (5 x 10⁶/10 ml medium) were seeded in a 10-cm tissue culture plate and incubated in medium containing 10% fetal bovine serum for 24 h. The cells were extensively washed with phosphate-buffered saline and then further grown in 10 ml of serum-free medium for 48 h.

The serum-free culture supernatants were withdrawn and sequentially centrifuged at 800 x g and 18,000 x g. Next, 2-ml aliquots were concentrated by using Centricon 30 concentrators (Amicon) and mixed with sodium dodecyl sulfate sample buffer. Electrophoresis was carried out by the method of Laemmli in 0.5 mg/ml type IV collagen-embedded polyacrylamide gels. After electrophoresis, gels were rinsed twice in 2.5% Triton X-100, 50 mM Tris-HC1, pH 7.5, and incubated at 37`C for 16 h in 0.15 M NaCl, 10 mM CaCl₂, 50 mM Tris-HCl, pH 7.5, containing 0.05% NaN₃. Gels were stained with 0.005% Coomassie blue and destained in 10% isopropanol and 10% acetic acid, and type IV collagenolytic enzymes were detected as transparent bands on slab gels.

### Results

### Human Colon Carcinoma

The four HCC cell lines were assayed for type IV collagenolytic activity. During the first 24 h incubation, KM12C, KM12L1, KM12L4 and KM12SM degraded 0.38, 0.62, 0.75, and 0.70 ug of type IV collagen, respectively. A similar degradation tendency for type IV collagen was observed at 48 h. During this period, KM12L4 and KM12SM degraded more type IV collagen than did KM12C and KM12L1 (1.25 and 1.20 versus 0.69 and 0.85 ug/10⁴ cells, respectively). The background release was 0.11 ug of type IV collagen degraded by serum proteases. The relationship between type IV collagenolytic activity and metastatic potential of the colon carcinoma cell lines was examined by comparing the type IV collagenolytic activity with the median number of hepatic metastases produced by cells injected into the spleen. After subtracting the background release, the amounts of type IV collagen degraded by colon carcinoma cells during the 24 h and 48 h incubations were plotted against the number of liver tumor colonies. The correlation coefficiency (r) and probability (P) are as follows: 24 h, r = 0.809, P = 0.1914; 40 h, r = 0.985, P = 0.0148.

The metastatic potential and type IV collagenolytic activity were also analyzed by the paired group t test. A significant difference between the low metastatic KM12C cells and the high metastatic KM12L4 and KM12SM cells (P<0.05) was found. The serum-free conditioned media were analyzed by enzymography to identify the proteinases responsible for type IV collagenolysis. The active proteinases of Mᵣ 98,000, 92,000, 80,000, 68,000, and 64,000 were shown as transparent bands on a type IV collagen-embeded polyacrylamide gel after Coomassie blue staining, with the 92 and 68 kd species being the most widely apparent in all of the lines (Figure 5). Relative enzyme activity, measured by densitometry, were 1.00, 3.90, 4.33, and 3.89 for KM12C, KM12L1, KM12L4, and KM12SM, respectively. A proteinase of Mᵣ 68,000 was only detected in the media conditioned by highly metastatic cell lines such KM12L4 and KM12SM. The type IV collagenolytic activities of the HCC cell lines therefore correlated with their potential to produce experimental hepatic metastases after intrasplenic implantation.

### Human Breast Cancer Malignant Melanoma Renal Cell Carcinoma, Teratocarcinoma, and Astrocytoma Lines

Similar results were obtained in studies employing human malignant melanoma cell (MMC), human breast cancer renal cell carcinoma, teratocarcinoma, and astrocytoma lines. Well known MMC lines, such as A375 (ATCC CRL1619), A375M, and Hs294T (ATCC HTB140), a breast cancer line MCF7 (ATCC HTB22) and astrocytoma lines, such as D54MG, U373MG, and U251MG, were obtained from various sources and tested as described above. Human renal carcinoma lines SN series were established by in vitro cultures of the primary tumor tissues (SN12C) or its transplate in the kidney of a nude mouse (SN12K), and selected for metastasis from the kidney to the liver (SN12L1) or the lung (SN12M6, SN12M7) in nude mice (15). Human PA-1 cells at four stages of tumor progression established by Tainsky et al. (16) were also tested for 88/92 kd metalloproteinase activity. These PA-1 cells includes 1) nontumorigenic, 2) preneoplastic and susceptible to transformation by single oncogenes, 3) tumorigenic, and 4) metastatic cells. In particular, these lines were employed to prepare conditioned media as described above for the HCC lines. The results demonstrated the appearance of the 88/92 kd enzyme in the media conditioned by all lines tested except for U373MG and U251MG cells. Furthermore, the enzyme levels were found to correlate with the tumorigenicity, invasiveness, or metastatic potential of the respective line. Among those cell lines the highly metastatic renal cell carcinoma cells (SN12L1, SN12M7) expressed the highest enzyme activities.

### Human Malignant Breast Cancer Patients

Sera obtained from a series of malignant breast cancer patients were tested for the presence of the 88/92 kd metalloproteinase by zymography. In all more than 30 breast cancer patients were tested. The samples used in this preliminary study were collected 4 to 6 years ago and stored at -20`C. Various of the results are shown in Figure 6, which demonstrate that of 12 of the patient's sera tested in the study shown, 9 tested positive for the 88/92 kd enzyme. The samples which did not exhibit the enzyme were probably inactivated during the storage.

### EXAMPLE III

### Partial Purification of the 88/92 kd Metalloproteinase from Rat and Human Sources

Studies have also been conducted wherein it has been determined that the 88/92 kd metalloproteinase can be readily obtained in useful amounts from conditioned media of either human or rat cells, such as the cell lines discussed above. In particular, the high molecular weight type IV collagenase has been isolated and partially purified through the use of both MTLn3 tumor-bearing rat serum, as well as from serum-free media conditioned by human breast cancer MCF7 cells. In general, where one desires to obtain larger quantities of the collagenase, one will desire to employ the conditioned media approach, which is also applicable to isolation of the enzyme from rat sources. The partial purification procedure employed heparin-Sepharose chromatography, in conjunction with gel filtration on column material such as Sephacryl S-200 or Sepharose CL-6B columns. Using such an approach, the inventors have found that an overall purification of on the order of 1 to 200 can routinely be achieved. Subsequent analyses of the partially purified material by SDS-PAGE techniques reveal that the material is more than 30% pure.

For isolation from rat serum, the following technique was employed. A rat received 1 X 10⁶ MTLn3 cells injected into the mammary fat pad. Approximately 30 days following subcutaneously injection of cells, the rat was sacrificed and the serum from the rat collected. Approximately 2 ml of sera was passed on a heparin-Separose column (bed volume 10 ml), the serum enzyme being retained on the column in a 0.15 M sodium chloride, 10 mM phosphate buffer at pH 7.2. The column was eluted with a gradient of 0.15-1.0 M sodium chloride and 10 mM phosphate buffer, pH 7.2. The active fractions eluted at a concentration range of 0.3-0.5 M sodium chloride. These fractions were collected and dialyzed against 0.1% sodium dodecyl sulfate in Dulbecco's phosphate-buffered saline, pH 7.2, for 3 h length of time and two changes of buffer. The dialyzed material was then concentrated and about 1 ml loaded onto a Sephacryl S-200 column (1 x 120cm) equilibrated and eluted with the same buffer. The enzyme activity remained in the void volume of this column.

The enzyme fraction collected off of the S-200 column was further purified by Sepharose CL-6B gel chromatography, using Tris buffer. The enzyme was eluted in fractions of K_{**av**} 0.4-0.5 in 50 mM Tris-HCl, 0.15 M sodium chloride, pH 7.5, containing 0.1% sodium dodecyl sulfate. The fractions exhibiting activity on zymograms were pooled and the buffer was replaced with 50 mM Tris-HCl, 0.2 M sodium chloride, 10 mM calcium chloride, 1 mM zinc chloride, pH 7.5, containing 0.5% Brij 35, and the fractions were stored at 4`C.

Partially purified fractions were tested for activity using a collagenolytic assay as described by Nakajima et al. (5). In general, suspensions of [³ H]-acetylated collagens were incubated at 27`C with the partially purified enzyme in 50 mM Tris-HCl, 0.1 M sodium chloride, 10 mM calcium chloride, pH 7.5, containing 0.05% Brij35, type IV collagen was cleaved into smaller fragments, but type I collagen was not degraded. Thus, the high molecular type IV collagenase has a preference for type IV collagen and probably gelatin. The results of these experiments demonstrated that the enzyme had been isolated and partially purified.

The high molecular weight type IV collagenase was also purified from rat sources using the serum-free medium conditioned by MTF7.T35.3 cells using the same procedures, with the serum-free media being prepared as described above in Example I.

From the foregoing studies it became apparent to the inventors that metalloproteinases having molecular weight of on the order of 88-92 kd capable of degrading type IV collagen and gelatin should prove to be useful markers for assessing the potential for tumor metastasis and for detecting a recurrence and monitoring patients that have had their primary tumors removed.

### EXAMPLE IV

### Preparation of Monoclonal Antibodies Against the 88/92 kd Metalloproteinase

Monoclonal antibodies are produced by the following methods: The partially purified rat enzyme is subjected to polyacrylamide cell electrophoresis in the presence of sodium dodecyl sulfate and the enzyme band is isolated therefrom, e.g., by excision of a gel slice, and electro-eluted. The purified enzyme is made into an emulsion with Freund's complete adjuvant and subcutaneously injected into BALB/c mice (200 ug antigen/mouse). The mice are boosted every two weeks i.p. with immunogen and Freund's complete adjuvant. After the third immunization the injection is administered intravenously without adjuvant. The mice are sacrificed 3-4 days after the last immunization, and their spleen is removed asceptically.

The spleen is gently teased over a screen to obtain a single cell suspension, followed by harvesting and washing of the lymphocytes. The splenocytes and P3X myeloma cells (ATCC CRL 1580) are mixed at a ratio of 1:1, pelleted by centrifugation, and polyethylene glycol added over a period of one minute with constant agitation at 37`C. After further stirring, medium is added and cells then washed. The resuspended fusion mixture is aliquoted into 96 well microtiter plates. Feeder cells prepared from new born mouse thymus are used at the initial low densities of the hybrids. The cells are then repeatedly fed HAT medium beginning the next day, and after 1-2 weeks, if growth is substantial, the supernatant media is screened for antibody production using ELISA.

The antibody specificity is checked by the immuno-blotting analysis described below. The resulting positive hybridomas are grown in defined serum-free medium which will allow easier purification of the monoclonal antibodies. The monoclonal antibodies are purified by ammonium sulfate precipitation and chromatography over protein A-agarose (Affi-Gel Protein A MAPS II Kit, Bio-Rad) and Sephadex G-25 columns.

### EXAMPLE V

### Immunologic Test For the 88/92 kd Metalloproteinase

### ELISA Inhibition Test

In the enzyme assays of sera and other body fluids, ELISA techniques will preferably be employed. For preparation of the serum sample, blood is withdrawn by venipuncture without anticoagulant and allowed to clot for about 1 h at 22`C. The clotted sample is centrifuged at 4`C for 10 min at 800 x g and for 15 min at 1,600 x g. The resultant sera is divided into small aliquots and snap-frozen in liquid nitrogen, and then maintained at*I902* -80`*I903*C until analyzed. A micro ELISA inhibition test will preferably be employed for serodiagnosis of cancer (see, e.g., ref. 26).

A proposed immunologic screening method is as follows. Microtest trays are coated with about 10 ug/ml 88/92 kd metalloproteinase in 0.1 M sodium bicarbonate buffer (pH 9.6) and incubated overnight at 4`C. The trays are blocked by the addition of 1% BSA in bicarbonate buffer for 1 h at 37`C. The wells are aspirated, and the trays are frozen at -76`C until ready for use. A 3 ul aliquot of serum from a cancer patient or a normal donor is added to each well, and then antibody solution (3 ul) diluted appropriately in 0.05% Tween-20 in 1% BSA in PBS, pH 7.4 is added. The trays are incubated for 2 h at 37`C, washed twice with 0.05% Tween-20 in PBS, and aspirated. Anti-mouse immunoglobulin conjugated to horse radish peroxidase solution (0.1 ug) in PBS containing 1% BSA is added to each well, and then the trays incubated for 1 h at 37`C. After washing twice with 0.05% Tween-20 in PBS, 5 ul of O-phenylenediamine substrate is added and incubated for 15 min. The reaction is terminated by the addition of 5 ul of 2.5 M sulfuric acid.

The optical density is measured with a microplate reader, such as a Titertek Multiskan (Flow Labs.). Percent inhibition is calculated for each OD value with use of the following formula: % = (1-OD/Pavg) x 100, where % = percent of inhibition, Pavg = average OD reading of the control sera. The inhibition of binding of antibodies on a linear scale is plotted against the concentration of purified 88/92 kd metalloproteinase added on a log scale. Using this standard curve, the concentration of the 88/92 kd metalloproteinase in serum can be determined. The relationship between serum 88/92 kd metalloproteinase levels and stages of disease can be studied by examining the patient records.

### Immunoblotting

An immunoblotting technique is employed to detect enzymes present in cells and tissues. It is believed that this technique will allow one to determine the amount of 88/92 kd metalloproteinase in active and inactive forms. Proenzymes and partial cleavage products may be detected. Immunoreactive proteins are visualized as bands at certain molecular weights and the quantitative analysis of positive protein bands is performed by densitometric scanning.

Equal aliquots (100 ug protein) of cell extracts or tissue extracts are subjected to SDS-polyacrylamide gel electrophoresis on a 5-15% gradient gel or 7.5% gel. The proteins are then electroblotted from the gel to nitro-cellulose filter paper (0.1 um pore size) for 2 h at 250 mA in chilled transfer buffer containing 20 mM Tris, 10 mM glycine, pH 9.15, using an electroblotting apparatus. The blots on the nitrocellulose paper are fixed in 50% aqueous isopropanol for 30 min, then rinsed in TST buffer (0.1% Tween 20, 200 mM NaCl, in 20 mM Tris-HCl, pH 7.5). The paper is then incubated for 3 h at 37`C in TST buffer containing 3% BSA, followed by a 3 h incubation with biotinylated antibodies at an appropriate concentration.

After incubation, the paper is rinsed three times in the same buffer and incubated in 5 mM HEPES, 200 mM NaCl, 0.1% Tween 20, pH 7.4 (HST) containing Streptoavidin-alkaline phosphatase for 1 h at 25`C. The paper will be washed three times with TST and three times with water, and developed with 0.16 mg/ml 5-bromo-4-chrolo-3-indoyl phosphate and 0.33 mg/ml Nitroblue tetrazolium in 0.1M NaCl, 50 mM MgCl₂, 0.1 M Tris-HCl, pH 9.5. The paper will be washed several times with water to stop the reaction, and air-dried. The positive bands which appear on the nitrocellulose paper are analyzed by densitometric scanning, e.g., with video-enhanced computer imaging and analysis.

It may also prove appropriate to use dot blot techniques for simple quantitative analysis of 88/92 kd metalloproteinase present in tissue and serum samples. In this technique, tissues are homogenized and solubilized with 0.1% SDS, 0.1% Triton X-100, 0.1% sodium chorate, 5 mM NEM, 1 mM PMSF in 0.15 M NaCl, 50 mM Tris-HCl buffer, pH 7.5, and centrifuged at 30,000 x g for 30 min. The supernatants are blotted on a nitrocellulose paper using a Bio-Dot microfiltration apparatus (Bio Rad). The serum samples are diluted with 0.1% SDS in phosphate buffered saline 10-fold and blotted on a nitrocellulose paper. Quantitative detection of antigen is performed as described above.

### Immunoperoxidase and Immunofluorescence Labeling

Studies on enzyme production and localization in various tissues and cells will be performed by immuno-staining techniques. To investigate differences between primary tumors and their metastases, and the heterogeneity of heparanase production among malignant cells in tumor tissues immunoperoxidase and immunofluorescence labeling will be used. The results will be also compared with the stages of disease referred to in the patient records.

In immunostaing, paraffin sections and frozen sections of tumor tissues can be stained by immuno-peroxidase techniques using purified antibodies and the Vectastain ABC kit (Vector Laboratories, Burlingame, CA) or goat anti-rabbit or mouse IgG conjugated with horse radish peroxidase. Immunoperoxidase staining is conducted according to the manufacturer's instructions, e.g., as provided by Vector Laboratories, and the stained specimens are examined on a light microscope. Immunoperoxidase staining can also be employed to examine intracellular and cell surface localization of 88/92 kd metalloproteinase. To see intracellular localization of 88/92 kd metalloproteinase, cells are permeabilized by acetone treatment prior to staining.

An alternate method, immunofluorescence labeling, is performed by the indirect immunostaining technique described using affinity-purified 88/92 kd metalloproteinase antibody (primary antibody) and F(ab')**₂** fluorescein-conjugated antibody fragments specific for the primary antibody (secondary antibody). The immunofluorescence-labeled specimens is examined on a UV-equipped light microscope (fluorescence microscope).

### REFERENCES

The references listed below are hereby incorporated by reference.
1. Nicolson, G.L. (1982), Cancer metastasis: Organ colonization and the cell-surface properties of malignant cells, Biochim. Biophys. Acta, 695:113-176.
2. Nicolson, G.L. (1988), Organ specificity of tumor metastasis: role of preferential adhesion, invasion and growth of malignant cells at specific sites. Cancer Metastasis Rev., 7:143-188.
3. Liotta et al. (1979), Preferential digestion of basement membrane collagen by an enzyme derived from a metastatic murine tumor, Proc. Natl. Acad. Sci. USA, 76:2268-2272.
4. Liotta et al. (1980), Metastatic potential correlates with enzymatic degradation of basement membrane collagen, Nature, 284:67-68.
5. Nakajima et al. (1987), Degradation of basement membrane type IV collagen and lung subendothelial matrix by rat mammary adenocarcinoma cell clones of differing metastatic potentials, Cancer Res., 47:4869-4876.
6. Salo et al. (1983), Purification and characterization of a murine basement membrane collagen-degrading enzyme secreted by metastatic tumor cells, J. Biol. Chem., 258:3058-3062.
7. Garbisa et al. (1986), Transient expression of type IV collagenolytic metalloproteinase by human mononuclear phagocytes, J. Biol. Chem., 261:2369-2375.
8. Collier et al. (1988), H-ras oncogene-transformed human bronchial epithelial cells (TBE-1) secrete a single metalloprotease capable of degrading basement membrane collagen, J. Biol. Chem., 263:6579-6587.
9. Nakajima et al. (1987), Basement membrane degradative enzymes and tumor metastasis, Cancer Bulletin, 39:142-149.
10. Pietras et al. (1979), Elevated serum cathepsin B1-like activity in women with neoplastic disease, Gynecol Oncol., 7:1-17.
11. Neri et al. (1982), Development and biologic properties of malignant cell sublines and clones of a spontaneously metastasizing rat mammary adenocarcinoma, J. Natl. Cancer Inst., 68:507-517.
12. Welch et al. (1983), Comparison of "spontaneous" and "experimental" metastasis using rat 13762 mammary adeno-carcinoma metastatic clones, Invasion Metastasis, 3:65-80.
13. Morikawa et al. (1988), In vivo selection of highly metastatic cells from surgical specimens of different primary human colon carcinomas implanted into nude mice, Cancer Res., 48:1943-1948.
14. Morikawa et al. (1988), Influence of organ environment on the growth, selection, and metastasis of human colon carcinoma cells in nude mice, Cancer Res., 48:6863-6871.
15. Naito et al. (1986), Growth and metastasis of tumor cells isolated from a human renal cell carcinoma implanted into different organ of nude mice, Cancer Res., 46:4109-4115.
16. Tainsky et al. (1988), PA-1, a human cell model for multistage carcinogenesis: oncogenes and other factors, Anticancer Res., 8:899-914.
17. Ridder et al. (1987), Invasiveness of human glioma cell lines in vitro: relation to tumorigenicity in athymic nude mice, Acta Neuropathol. (Berl), 72:207-213.
18. Edited by Langone and Van Vunakis (1986), selected applications of monoclonal antibodies, Methods in Enzymology, 121:695-867.
19. Hsu et al. (1981), A comparative study of the peroxidase-antiperosidase method and avidin-biotin complex method for studying polypeptide hormones with radioimmunoassay antibodies, Am. J. Clin. Pathol., 75:734-738.
20. Poste et al. (1988), The pathogenesis of cancer metastasis, Nature, (Lond.), 283:139-146.
21. Nicolson et al. (1983), Tumor implantation and invasion of metastatic sites, Int. Rev. Exp. Pathol., 25:77-81.
22. Liotta et al. (1986), Biochemical interactions of tumor cells with the basement membrane, Annul. Rev. Biochem., 55:1037-1057.
23. Tryggvason et al. (1987), Proteolytic degradation of extracellular matrix in tumor invasion, Biochim. Biophys. Acta, 907:191-217.
24. Heussen et al. (1980), Electrophoretic analysis of plasminogen activators in polyacrylamide gels containing sodium dodecyl sulfate and copolymerized substrates, Anal. Biochem., 102:196-202.
25. Laemmli (1970), Cleavage of structural proteins during the assembly of the head of bacteriophage T4, Nature (Lond.), 227:680-685.
26. Katz et al. (1981), Detection of monoclonal antibodies for tumor diagnosis with the use of inhibition of micro-enzyme-linked immunosorbent assay, J. Natl. Cancer Inst., 74:335-339.

## Claims (Claims for the following Contracting State(s): AT, BE, CH, DE, DK, FR, GB, IT, LI, LU, NL, SE)

1. A gelatinolytic and type IV collagenolytic rat metalloproteinase exhibiting a molecular weight of between about 88 and 92 kilodaltons upon being subjected to sodium dodecyl sulfate-polyacrylamide gel electrophoresis.

2. The metalloproteinase of claim 1, obtainable by growth of metastatic mammary carcinoma cells characterized by an ability to secrete the metalloproteinase.

3. The metallproteinase of claim 1, obtainable by growth of colon carcinoma, renal cell carcinoma, malignant melanoma, teratocarcinoma, or astrocytoma cells, each characterized by an ability to secrete the metalloproteinase.

4. A method for providing a metallproteinase exhibiting a molecular weight of between about 88 and 92 kilodaltons upon being subjected to sodium dodecyl sulfate-polyacrylamide gel electrophoresis, wherein said method comprises the following steps
(a) culturing a human tumor cell which secretes said metalloproteinase to form a conditioned medium,
(b) subjecting said conditioned medium to heparin-sepharose chromatography fractionation,
(c) purifying active fractions by gel filtration chromatography using Sephacryl S-200 or Sepharose CL-6 B.

5. The method of claim 4, wherein the human tumor cells comprises metastatic mammary carcinoma cells, colon carcinoma cells or a malignant melanoma cells, renal cell carcinoma cells, astrocytoma cells, or teratocarcinoma cells.

6. The method of claim 4, wherein the human tumor cell comprises HS294T malignant melanoma cells.

7. A method for providing a metalloproteinase in accordance with claim 1, the method comprising preparing the metalloproteinase from the serum of a rat bearing a metastatic mammary adenocarcinoma.

8. The method of claim 7, wherein the metastatic mammary adenocarcinoma comprises a metastatic clone of 13762NF mammary adenocarcinoma cells.

9. A method for testing for human metastatic disease or tumor recurrence in a patient suspected of having such a disease, the method comprising testing for the presence of a metalloproteinase obtainable in accordance with claim 4, in a clinical sample obtainable from the patient, wherein testing for the presence of the metalloproteinase includes the steps of:
- subjecting a clinical sample to electrophoresis in a substrate-embedded gel to separate proteins within the sample;
- incubating the gel under conditions appropriate to allow any metalloproteinase as defined by claim 1 which may be present in the gel to lyse the substrate; and
- identifying the presence of such a metalloproteinase in the gel following such an incubation, the presence of the metalloproteinase being indicative of the presence of the disease,
- wherein identifying the presence of such a metalloproteinase includes comparing any metalloproteinase identified with a standard metalloproteinase as defined by claim 4.

10. The method of claim 9, wherein the substrate comprises type I collagen, type IV collagen or gelatin.

11. The method of claim 9, wherein the gel comprises a polyacrylamide gel.

12. The method of claim 9, wherein testing for the presence of the metalloproteinase comprises testing the sample immunologically through the use of an antibody against the metalloproteinase.

13. A kit for testing for the presence of a metalloproteinase in a biological sample, the kit comprising an antibody directed against the metalloproteinase of claim 1 and means for detecting an immunoreaction between such antibodies and the metallproteinase.

14. A method for providing a gelatinolytic and type IV collagenolytic metalloproteinase exhibiting a molecular weight of between about 88 and 92 kilodaltons upon being subjected to sodium dodecyl sulfate - polyacrylamide gel electrophoresis, wherein said method comprises the following steps
(a) culturing a rat tumor cell which secretes said metalloproteinase to form a conditioned medium,
(b) subjecting said conditioned medium to heparin-sepharose chromatography fractionation , and
(c) purifying active fractions by gel filtration chromatography,
wherein said conditioned medium is the serum of a rat bearing a metastatic mammary adenocarcinoma

15. The method of claim 14, wherein Sephacryl S-200 or Sepharose CL-6B is used for gel filtration chromatography.

16. The method of claim 14 or 15 wherein the metastatic mammary adenocarcinoma comprises a metastatic clone of 13762 NF mammary adenocarcinoma cells.

## Claims (Claims for the following Contracting State(s): ES)

1. A method for providing a metallproteinase exhibiting a molecular weight of between about 88 and 92 kilodaltons upon being subjected to sodium dodecyl sulfate-polyacrylamide gel electrophoresis, wherein said method comprises the following steps
(a) culturing a human tumor cell which secretes said metalloproteinase to form a conditioned medium,
(b) subjecting said conditioned medium to heparin-sepharose chromatography fractionation,
(c) purifying active fractions by gel filtration chromatography using Sephacryl S-200 or Sepharose CL-6B.

2. The method of claim 1, wherein the human tumor cells comprises metastatic mammary carcinoma cells, colon carcinoma cells or malignant melanoma cells, renal cell carcinoma cells, astrocytoma cells, or teratocarcinoma cells.

3. The method of claim 2, wherein said metastatic mammary carcinoma cells are characterized by an ability to secrete the metalloproteinase.

4. The method of claim 1, wherein the human tumor cell comprises HS294T malignant melanoma cells.

5. A method for providing a gelatinolytic and type IV collagenolytic rat metalloproteinase exhibiting a molecular weight of between about 88 and 92 kilodaltons upon being subjected to sodium dodecyl sulfate-polyacrylamide gel electrophoresis, the method comprising preparing the metalloproteinase from the serum of a rat bearing a metastatic mammary adenocarcinoma.

6. The method of claim 5, wherein the metastatic mammary adenocarcinoma comprises a metastatic clone of 13762NF mammary adenocarcinoma cells.

7. A method for testing for human metastatic disease or tumor recurrence in a patient suspected of having such a disease, the method comprising testing for the presence of a metalloproteinase obtainable in accordance with claim 1, in a clinical sample obtainable from the patient, wherein testing for the presence of the metalloproteinase includes the steps of:
- subjecting a clinical sample to electrophoresis in a substrate-embedded gel to separate proteins within the sample;
- incubating the gel under conditions appropriate to allow any gelatinolytic and type IV collagenolytic rat metalloproteinase, exhibiting a molecular weight of between about 88 and 92 kilodaltons upon being subjected to sodium dodecyl sulfate-polyacrylamide gel electrophoresis, which may be present in the gel to lyse the substrate; and
- identifying the presence of such a metalloproteinase in the gel following such an incubation, the presence of the metalloproteinase being indicative of the presence of the disease,
- wherein identifying the presence of such a metalloproteinase includes comparing any metalloproteinase identified with a standard metalloproteinase as defined by claim 1.

8. The method of claim 7, wherein the substrate comprises type I collagen, type IV collagen or gelatin.

9. The method of claim 7, wherein the gel comprises a polyacrylamide gel.

10. The method of claim 7, wherein testing for the presence of the metalloproteinase comprises testing the sample immunologically through the use of an antibody against the metalloproteinase.

11. A process for preparing a kit for testing for the presence of a metalloproteinase in a biological sample, the kit comprising an antibody directed against a gelatinolytic and type IV collagenolytic rat metalloproteinase exhibiting a molecular weight of between about 88 and 92 kilodaltons upon being subjected to sodium dodecyl sulfate-polyacrylamide gel electrophoresis, and means for detecting an immunoreaction between such antibodies and the metallproteinase.

12. A method for providing a gelatinolytic and type IV collagenolytic metalloproteinase exhibiting a molecular weight of between about 88 and 92 kilodaltons upon being subjected to sodium dodecyl sulfate-polyacrylamide gel electrophoresis, wherein said method comprises the following steps
(a) culturing a rat tumor cell which secretes said metalloproteinase to form a conditioned medium,
(b) subjecting said conditioned medium to heparin-sepharose chromatography fractionation, and
(c) purifying active fractions by gel filtration chromatography,
wherein said conditioned medium is the serum of a rat bearing a metastatic mammary adenocarcinoma.

13. The method of claim 12, wherein Sephacryl S-200 or Sepharose CL-6B is used for gel filtration chromatography.

14. The method of claim 12 or 13, wherein the metastatic mammary adenocarcinoma comprises a metastatic clone of 13762NF mammary adenocarcinoma cells.

15. A kit for testing for the presence of a metalloproteinase in a biological sample, the kit comprising an antibody directed against a gelatinolytic and type IV collagenolytic rat metalloproteinase exhibiting a molecular weight of between about 88 and 92 kilodaltons upon being subjected to sodium dodecyl sulfate-polyacrylamide gel electrophoresis, and means for detecting an immunoreaction between such antibodies and the metalloproteinase.

## Patentansprüche (Patentansprüche für folgende(n) Vertragsstaat(en): AT, BE, CH, DE, DK, FR, GB, IT, LI, LU, NL, SE)

1. Gelatinolytische und Typ IV-kollagenolytische Ratten-Metallproteinase mit einem Molekulargewicht zwischen etwa 88 und 92 Kilodalton bei der Natriumdodecylsulfat-Polyacrylamidgel-Elektrophorese.

2. Metallproteinase nach Anspruch 1, erhältlich durch Züchten von metastatischen Mamma-Karzinomzellen, charakterisiert durch eine Fähigkeit zur Sekretion der Metallproteinase.

3. Metallproteinase nach Anspruch 1, erhältlich durch Züchtung von Kolon-Karzinom-, Nieren-Zellkarzinom-, malignen Melanom-, Teratokarzinom- oder Astrocytom-Zellen, die jeweils durch eine Fähigkeit zur Sekretion der Metallproteinase charakterisiert sind.

4. Verfahren zur Bereitstellung einer Metallproteinase mit einem Molekulargewicht zwischen etwa 88 und 92 Kilodalton bei der Natriumdodecylsulfat-Polyacrylamidgel-Elektrophorese, wobei das Verfahren folgende Stufen umfaßt:
(a) eine humane Tumorzelle, die die Metallproteinase sekretiert, wird zur Bildung eines konditionierten Mediums gezüchtet,
(b) das konditionierte Medium wird der Heparin-Sepharose-Chromatographiefraktionierung unterworfen, und
(c) die aktiven Fraktionen werden unter Verwendung von Sephacryl S-200 oder Sepharose CL-6 B durch Gelfiltrationschromatographie gereinigt.

5. Verfahren nach Anspruch 4, wobei die humanen Tumorzellen metastatische Mamma-Karzinomzellen, Kolon-Karzinomzellen oder maligne Melanomzellen, Nieren-Zellkarzinom-Zellen, Astrocytom-Zellen oder Teratokarzinom-Zellen umfassen.

6. Verfahren nach Anspruch 4, wobei die humane Tumorzelle maligne HS294T-Melanomzellen umfaßt.

7. Verfahren zur Bereitstellung einer Metallproteinase gemäß Anspruch 1, wobei das Verfahren die Herstellung einer Metallproteinase aus dem Serum einer Ratte mit einem metastatischen Mamma-Adenokarzinom umfaßt.

8. Verfahren nach Anspruch 7, wobei das metastatische Mamma-Adenokarzinom einen metastatischen Klon von 13762NF-Mamma-Adenokarzinomzellen umfaßt .

9. Verfahren zum Testen einer humanen metastatischen Erkrankung oder von Tumorrezidiven in einem Patienten, bei dem ein Verdacht auf eine derartige Erkrankung besteht, wobei das Verfahren den Test auf die Anwesenheit einer gemäß Anspruch 4 erhältlichen Metallproteinase in einer klinischen Probe des Patienten umfaßt, wobei der Test auf die Anwesenheit der Metallproteinase folgende Stufen umfaßt:
- eine klinische Probe wird der Elektrophorese in einem Gel mit eingebettetem Substrat unterworfen, um die Proteine innerhalb der Probe aufzutrennen;
- das Gel wird unter Bedingungen inkubiert, die es einer Metallproteinase gemäß der Definition in Anspruch 1, die im Gel vorhanden sein kann, ermöglichen, das Substrat zu lysieren; und
- die Anwesenheit einer derartigen Metallproteinase im Gel wird im Anschluß an eine derartige Inkubation identifiziert, wobei die Anwesenheit der Metallproteinase einen Indikator für das Vorliegen der Erkrankung darstellt,
- wobei die Indentifikation der Anwesenheit einer derartigen Metallproteinase den Vergleich einer identifizierten Metallproteinase mit einer Standard-Metallproteinase nach Anspruch 4 umfaßt.

10. Verfahren nach Anspruch 9, wobei das Substrat Typ I-Kollagen, Typ IV-Kollagen oder Gelatine umfaßt.

11. Verfahren nach Anspruch 9, wobei das Gel ein Polyacrylamidgel umfaßt.

12. Verfahren nach Anspruch 9, wobei der Test auf die Anwesenheit der Metallproteinase den immunologischen Test der Probe unter Verwendung eines Antikörpers gegen die Metallproteinase umfaßt.

13. Kit zum Test auf die Anwesenheit einer Metallproteinase in einer biologischen Probe, wobei das Kit einen Antikörper gegen die Metallproteinase von Anspruch 1 und eine Einrichtung zum Nachweis einer Immunoreaktion zwischen derartigem Antikörper und der Metallproteinase umfaßt.

14. Verfahren zur Bereitstellung einer gelatinolytischen und Typ IV-kollagenolytischen Metallproteinase, die ein Molekulargewicht zwischen etwa 88 und 92 Kilodalton bei der Natriumdodecylsulfat-Polyacrylamidgel-Elektrophorese aufweist, wobei das Verfahren folgende Stufen umfaßt:
(a) eine Ratten-Tumorzelle, die die Metallproteinase sekretiert, wird zur Bildung eines konditionierten Mediums gezüchtet,
(b) das konditionierte Medium wird der Heparin-Sepharose-Chromatographiefraktionierung unterworfen, und
(c) die aktiven Fraktionen werden durch Gelfiltrationschromatographie gereinigt,
wobei es sich beim konditionierten Medium um das Serum einer Ratte mit metastatischem Mamma-Adenokarzinon handelt.

15. Verfahren nach Anspruch 14, wobei Sephacryl S-200 oder Sepharose CL-6B für die Gelfiltrationschromatographie verwendet werden.

16. Verfahren nach Anspruch 14 oder 15, wobei das metastatische Mamma-Adenokarzinom einen metastatischen Klon von 13762NF-Mamma-Adenokarzinomzellen umfaßt.

## Patentansprüche (Patentansprüche für folgende(n) Vertragsstaat(en): ES)

1. Verfahren zur Bereitstellung einer Metallproteinase mit einem Molekulargewicht zwischen etwa 88 und 92 Kilodalton bei der Natriumdodecylsulfat-Polyacrylamidgel-Elektrophorese, wobei das Verfahren folgende Stufen umfaßt:
(a) eine humane Tumorzelle, die die Metallproteinase sekretiert, wird zur Bildung eines konditionierten Mediums gezüchtet,
(b) das konditionierte Medium wird der Heparin-Sepharose-Chromatographiefraktionierung unterworfen, und
(c) die aktiven Fraktionen werden unter Verwendung von Sephacryl S-200 oder Sepharose CL-6 B durch Gelfiltrationschromatographie gereinigt.

2. Verfahren nach Anspruch 1, wobei die humanen Tumorzellen metastatische Mamma-Karzinom-, Kolon-Karzinom-, maligne Melanom-, Nieren-Zellkarzinom-, Astrocytom- oder Teratokarzinom-Zellen umfassen.

3. Verfahren nach Anspruch 2, wobei die metastatischen Mamma-Karzinomzellen durch ihre Fähigkeit zur Sekretion der Metallproteinase charakterisiert sind.

4. Verfahren nach Anspruch 1, wobei die humanen Tumorzellen maligne HS294T-Melanomzellen umfassen.

5. Verfahren zur Bereitstellung einer gelatinolytischen und Typ IV-kollagenolytischen Ratten-Metallproteinase mit einem Molekulargewicht zwischen etwa 88 und 92 Kilodalton bei der Natriumdodecylsulfat-Polyacrylamidgel-Elektrophorese, wobei das Verfahren die Präparation der Metallproteinase aus dem Serum einer Ratte mit einem metastatischen Mamma-Adenokarzionom umfaßt.

6. Verfahren nach Anspruch 5, wobei das metastatische Mamma-Adenokarzionom einen metastischen Klon von 13762NF-Mamma-Adenokarzionomzellen umfaßt.

7. Verfahren zum Testen von humanen metastatische Erkrankungen oder von Tumorrezidiven in einem Patienten, bei dem ein Verdacht auf eine derartige Erkrankung besteht, wobei das Verfahren den Test auf die Anwesenheit einer gemäß Anspruch 1 erhältlichen Metallproteinase in einer klinischen Probe des Patienten umfaßt, wobei der Test auf die Anwesenheit der Metallproteinase folgende Stufen umfaßt:
- eine klinische Probe wird der Elektrophorese in einem Gel mit eingebettetem Substrat unterworfen, um die Proteine innerhalb der Probe aufzutrennen;
- das Gel wird unter Bedingungen inkubiert, die es einer beliebigen gelatinolytischen und Typ IV-kollagenolytischen Ratten-Metallproteinase mit einem Molekulargewicht zwischen etwa 88 und 92 Kilodalton bei der Natriumdodecylsulfat-Polyacrylamidgel-Elektrophorese, die im Gel vorhanden sein kann, ermöglichen, das Substrat zu lysieren und
- die Anwesenheit einer derartigen Metallproteinase im Gel wird im Anschluß an eine derartige Inkubation identifiziert, wobei die Anwesenheit der Metallproteinase einen Indikator für das Vorliegen der Erkrankung darstellt,
- wobei die Indentifikation der Anwesenheit einer derartigen Metallproteinase den Vergleich einer identifizierten Metallproteinase mit einer Standard-Metallproteinase nach Anspruch 1 umfaßt.

8. Verfahren nach Anspruch 7, wobei das Substrat Typ I-Kollagen, Typ IV-Kollagen oder Gelatine umfaßt.

9. Verfahren nach Anspruch 7, wobei das Gel ein Polyacrylamidgel umfaßt.

10. Verfahren nach Anspruch 7, wobei der Test auf die Anwesenheit der Metallproteinase den immunologischen Test der Probe unter Verwendung eines Antikörpers gegen die Metallproteinase umfaßt.

11. Verfahren zur Herstellung eines Kits zum Testen auf die Anwesenheit einer Metallproteinase in einer klinischen Probe, wobei das Kit einen Antikörper gegen eine gelatinolytische und Typ IV-kollagenolytische Ratten-Metallproteinase, die ein Molekulargewicht zwischen etwa 88 und 92 Kilodalton bei der Natriumdodecylsulfat-Polyacrylamidgel-Elektrophorese aufweist, und Mittel zum Nachweis einer Immunreaktion zwischen derartigen Antikörpern und der Metallproteinase umfaßt.

12. Verfahren zur Bereitstellung einer gelatinolytischen und Typ IV-kollagenolytischen Metallproteinase mit einem Molekulargewicht zwischen etwa 88 und 92 Kilodalton bei der Natriumdodecylsulfat-Polyacrylamidgel-Elektrophorese, wobei das Verfahren folgende Stufen umfaßt:
(a) eine Ratten-Tumorzelle, die die Metallproteinase sekretiert, wird zur Bildung eines konditionierten Mediums gezüchtet,
(b) das konditionierte Medium wird der Heparin-Sepharose-Chromatographiefraktionierung unterworfen, und
(c) die aktiven Fraktionen werden durch Gelfiltrationschromatographie gereinigt,
wobei es sich beim konditionierten Medium um das Serum einer Ratte mit metastatischem Mamma-Adenokarzinom handelt.

13. Verfahren nach Anspruch 12, wobei Sephacryl S-200 oder Sepharose CL-6B für die Gelfiltrationschromatographie verwendet werden.

14. Verfahren nach Anspruch 12 oder 13, wobei das metastatische Mamma-Adenokarzinom einen metastatischen Klon von 13762NF-Mamma-Adenokarzinomzellen umfaßt.

15. Kit zum Testen auf die Anwesenheit einer Metallproteinase in einer biologischen Probe, wobei das Kit einen Antikörper gegen eine gelatinolytische und Typ IV-kollagenolytische Ratten-Metallproteinase, die ein Molekulargewicht zwischen etwa 88 und 92 Kilodalton bei der Natriumdodecylsulfat-Polyacrylamidgel-Elektrophorese aufweist, und Mittel zum Nachweis einer Immunreaktion zwischen derartigen Antikörpern und der Metallproteinase umfaßt.

## Revendications (Revendications pour l'(les) Etat(s) contractant(s) suivant(s): AT, BE, CH, DE, DK, FR, GB, IT, LI, LU, NL, SE)

1. Métalloprotéinase de rat collagénolytique de type IV et gélatinolytique présentant un poids moléculaire d'environ 88 et 92 kilodaltons après avoir été soumis à une électrophorèse sur gel de sodium-dodécyl-sulfate-polyacrylamide.

2. Métalloprotéinase selon la revendication 1, pouvant être obtenue par croissance de cellules de carcinome mammaire métastasiques caractérisée par une capacité à secréter la métalloprotéinase.

3. Métalloprotéinase selon la revendication 1 pouvant être obtenue par croissance de cellules de carcinome de colon, de carcinome cellulaire rénal, de mélanome malin, de terato-carcinome, ou d'astrocytome, chacune caractérisée par une capacité à secréter la métalloprotéinase.

4. Procédé pour fournir une métalloprotéinase présentant un poids moléculaire d'entre environ 88 et 92 kd après avoir été soumis à une électrophorèse sur gel de sodium-dodécyl-sulfate-polyacrylamide, dans lequel ladite méthode consiste en les étapes suivantes
a) cultiver une cellule tumorale humaine qui secrète ladite métalloprotéinase pour former un milieu conditionné,
b) soumettre ledit milieu conditionné à un fractionnement par chromatographie d'héparine-Sépharose,
c) purifier les fractions actives par chromatographie en filtration sur gel en utilisant la Séphacryl-S-200 ou la Sépharose CL-6-V.

5. Procédé selon la revendication 4, dans lequel les cellules tumorales humaines comportent des cellules de carcinome mammaire métastasiques, des cellules de carcinome de côlon ou des cellules de mélanome malin, des cellules de carcinome cellulaires rénales, des cellules d'astrocytome, ou des cellules de tératocarcinomes.

6. Procédé selon la revendication 4, dans lequel la cellule tumorale humaine consiste en des cellules de mélanome malin HS294T.

7. Procédé pour fournir une métalloprotéinase en accord avec la revendication 1, le procédé consistant en la préparation de la métalloprotéinase à partir de sérum d'un rat portant un adénocarcinome mammaire métastasique.

8. Procédé selon la revendication 7, dans lequel l'adéno carcinome mammaire métastasique consiste en un clone métastasique de cellules d'adéno carcinome mammaire 13762NF.

9. Procédé pour tester la maladie métastasique humaine ou une récurrence tumorale dans un patient supposé avoir une telle maladie, le procédé consiste en un test de la presence d'une metalloproteinase pouvant être obtenue en accord avec la revendication 4, dans un échantillon clinique pouvant être obtenu à partir du patient, dans lequel le test pour la présence de la métalloprotéinase inclut les étapes :
- soumettre un échantillon clinique à l'électrophorèse dans un gel enrobé de substrat pour séparer les protéines au sein de l'échantillon,
- incuber le gel dans des conditions appropriées pour permettre à toutes les métalloprotéinases telles que définies par la revendication 1 qui pourraient être présentes dans le gel de lyser le substrat,
- identifier la présence d'une telle métalloprotéinase dans le gel suite à une telle incubation, la présence de la métalloprotéinase étant indicatrice de la présence de la maladie,
- dans lequel test l'identification de la présence d'une telle métalloprotéinase inclue la comparaison avec quelques métalloprotéinases que ce soit identifiées avec une metalloproteinase standard comme défini par la revendication 4.

10. Procédé selon la revendication 9, dans lequel le substrat consiste en du collagène de type I, du collagène de type IV ou de la gélatine.

11. Procédé selon la revendication 9, dans lequel le gel consiste en un gel de polyacrylamide.

12. Procédé selon la revendication 9, dans lequel le test pour la présence de la métalloprotéinase consiste en un test de l'échantillon de manière immunologique par le biais de l'utilisation d'un anticorps contre la métalloprotéinase.

13. Kit pour le test de la présence d'une metalloproteinase dans un échantillon biologique, le kit consistant en un anticorps dirigé contre la métalloprotéinase selon la revendication 1 et des moyens pour la détection d'une immunoréaction entre de tels anticorps et la métalloprotéinase.

14. Procédé pour fournir une métalloprotéinase gélatinolytique et collagénolytique de type IV présentant un poids moléculaire d'entre environ 88 et 92 kd après avoir été soumis à une électrophorèse sur gel de sodiumdodécyl-sulfate-polyacrylamide, dans lequel ladite méthode consiste en les étapes suivantes :
a) cultiver une cellule tumorale de rat qui secrète ladite métalloprotéinase pour former un milieu conditionné,
b) soumettre ledit milieu conditionné à un fractionnement par chromatographie d'héparine-Sépharose,
c) purifier les fractions actives par une chromatographie de filtration sur gel,
Dans lequel ledit milieu conditionné est le sérum d'un rat portant un adénocarcinome mammaire métastasique.

15. Procédé selon la revendication 14, dans lequel de la Séphacryl S-200 ou de la Sépharose CL-6B est utilisée pour la chromatographie de filtration sur gel.

16. Procédé selon la revendication 14 ou 15 dans lequel l'adénocarcinome mammaire métastasique consiste en un clone métastasique de cellules d'adénocarcinome mammaire 13762NF.

## Revendications (Revendications pour l'(les) Etat(s) contractant(s) suivant(s): ES)

1. Procédé pour fournir une métalloprotéinase présentant un poids moléculaire d'entre environ 88 et 92 kd après avoir été soumis à une électrophorèse sur gel de sodium-dodécyl-sulfate-polyacrylamide, dans lequel ledit procédé consiste en les étapes suivantes :
a) cultiver une cellule tumorale humaine qui secrète ladite métalloprotéinase pour former un milieu conditionné,
b) soumettre ledit milieu conditionné à un fractionnement en chromatographie héparine-Sépharose,
c) purifier les fractions actives par une chromatographie de filtration sur gel en utilisant de la Séphacryl-200 ou de la Sépharose CL-6B.

2. Procédé selon la revendication 1, dans lequel les cellules tumorales humaines consistent en des cellules de carcinome mammaire métastasique, des cellules de carcinome du côlon ou des cellules de mélanome malin, des cellules de carcinome cellulaire renal, des cellules d'astrocytome, ou des cellules de tératocarcinome.

3. Procédé selon la revendication 2, dans lequel lesdites cellules de carcinome mammaire métastasique sont caractérisées par une capacité à secréter la métalloprotéinase.

4. Procédé selon la revendication 1, dans lequel la cellule tumorale humaine consiste en des cellules de mélanome malin HS294T.

5. Procédé pour fournir une metalloproteinase de rat gélatinolytique et collagénolytique de type IV présentant un poids moléculaire d'entre environ 88 et 92 kd après avoir été soumis à une électrophorèse sur gel de sodium-dodécyl-sulfate-polyacrylamide, le procédé consistant en la preparation de la metalloproteinase a partir de sérum de rat portant un adénocarcinome mammaire métastasique.

6. Procédé selon la revendication 5, dans lequel l'adénocarcinome mammaire métastasique consiste en un clone métastasique de cellules d'adénocarcinome mammaire 13762NF.

7. Procédé pour tester la maladie métastasique humaine ou une récurrence tumorale chez un patient supposé avoir une telle maladie, le procédé consiste en un test de la présence d'une métalloprotéinase pouvant être obtenue en accord avec la revendication 1, dans un échantillon clinique pouvant être obtenu à partir du patient, dans lequel le test pour l'absence de la métalloprotéinase inclue les étapes :
- soumettre un échantillon clinique à une électrophorèse dans un gel enrobé de substrat pour séparer les protéines au sein de l'échantillon,
- incuber le gel dans des conditions appropriées pour permettre à n'importe laquelle des métalloprotéinases gélatinolytique et collagénolytique de type IV, de rat présentant un poids moléculaire d'entre environ 88 et 92 kd après avoir été soumis à une électrophorèse sur gel de sodium-dodécyl-sulfate-polyacrylamide, qui peut être présente dans le gel de lyser le substrat,
- identifier la présence d'une telle métalloprotéinase dans le gel suite à une telle incubation, la présence de la métalloprotéinase étant indicatrice de la présence de la maladie,
- dans lequel test l'identification de la présence d'une telle metalloproteinase inclue la comparaison de quelques métalloprotéinases que ce soit identifiées avec une métalloprotéinase standard comme défini par la revendication 1.

8. Procédé selon la revendication 7, dans lequel le substrat consiste en du collagène de type I, du collagène de type IV ou de la gélatine.

9. Procédé selon la revendication 7, dans lequel le gel consiste en un gel de polyacrylamide.

10. Procédé selon la revendication 7, dans lequel le test de la présence de la metalloproteinase consiste à tester l'échantillon de manière immunologique par le biais de l'utilisation d'un anticorps contre la métalloprotéinase.

11. Procédé pour la préparation d'un kit pour le test de la presence d'une métalloprotéinase dans un échantillon biologique, le kit consiste en un anticorps dirigé contre une métalloprotéinase gélatinolytique et collagénolytique de type IV de rat présentant un poids moléculaire d'entre environ 88 et 92 kd après avoir été soumis à une électrophorèse sur gel de sodium-dodécylsulfate-polyacrylamide, et des moyens pour détecter une immunoréaction entre de tels anticorps et la métalloprotéinase.

12. Procédé pour fournir une metalloproteinase gélatinolytique et collagénolytique de type IV, présentant un poids moléculaire d'entre environ 88 et 92 kd après avoir été soumis à une électrophorèse sur gel de sodiumdodécyl-sulfate-polyacrylamide, dans lequel ledit procédé consiste en les étapes suivantes :
a) cultiver une cellule tumorale de rat qui secrète ladite métalloprotéinase pour former un milieu conditionné,
b) soumettre ledit milieu conditionné à un fractionnement par chromatographie en héparine-Sépharose,
c) purifier les fractions actives par une chromatographie en filtration sur gel,
dans lequel ledit milieu conditionné et le serum d'un rat portant un adénocarcinome mammaire métastasique.

13. Procédé selon la revendication 12, dans lequel la Séphacryl-200 ou la Sépharose CL-6B est utilisée pour la chromatographie en filtration sur gel.

14. Procédé selon la revendication 12 ou 13, dans lequel l'adéno carcino mammaire métastasique consiste en un clone métastasique de cellules d'adénocarcinome mammaire 13762NF.

15. Kit pour le test de la présence d'une métalloprotéinase dans un échantillon biologique, le kit consiste en un anticorps dirigé contre une métalloprotéinase gélatinolytique et collagénolytique de type IV de rat présentant un poids moléculaire d'entre environ 88 et 92 kd après avoir été soumis à une électrophorèse sur gel de sodium-dodecyl-sulfate-polyacrylamide, et des moyens pour détecter une immunoréaction entre de tels anticorps et la métalloprotéinase.
